# EUROPEAN PATENT SPECIFICATION

(11) **EP 3 972 670 B1**
(45) Date of publication and mention of the grant of the patent: **16.09.2026**
(21) Application number: 20725717.1
(22) Date of filing: 18.05.2020
(51) Int. Cl.: A61M 5/142, A61M 5/145, A61M 5/172

(54) **DELIVERY DEVICE WITH SENSORLESS MOTOR CONTROL**
ABGABEVORRICHTUNG MIT SENSORLOSER MOTORSTEUERUNG
DISPOSITIF DE DISTRIBUTION AVEC UNE COMMANDE DE MOTEUR SANS CAPTEUR

(30) Priority: 23.05.2019 EP 19176233
(43) Date of publication of application: 30.03.2022
(73) Proprietor: Ypsomed AG, 3401 Burgdorf (CH)
(72) Inventor: RYTZ, Bernhard, 3436 Zollbrück (CH); MÜHLEMANN, Martin, 3625 Heiligenschwendi (CH)
(74) Representative: Meier Obertüfer, Jürg
(86) International application number: PCT/EP2020/063792
(87) International publication number: WO 2020/234229

(56) References cited:
- WO-A1-2019/008562
- US-A1- 2008 197 794
- US-A1- 2011 227 519
- US-A1- 2018 069 494
- WEIZI WANG ET AL: "Sensorless control technology for single phase BLDCM based on the winding time-sharing method", INDUSTRIAL ELECTRONICS SOCIETY, 2005. IECON 2005. 31ST ANNUAL CONFERENCE OF IEEE, IEEE, PISCATAWAY, NJ, USA, 6 November 2005 (2005-11-06), pages 1732 - 1736, XP010876231, ISBN: 978-0-7803-9252-6, DOI: 10.1109/IECON.2005.1569166

## Description

### FIELD OF THE INVENTION

The present invention relates to drug delivery systems for delivering, administering, injecting, infusing and/or dispensing liquids comprising a medicament or active ingredient. It departs from a delivery device with an electric motor for driving a piston in a drug reservoir towards a cannula attached to the reservoir.

### BACKGROUND OF THE INVENTION

A variety of diseases exists that require regular treatment by subcutaneous, intradermal, or intramuscular administration of a medicament. In response, a number of drug delivery devices has been developed to support a patient in conveniently and controllably delivering a precise amount of drug in a self-administration process. Delivery devices include injection devices that are removed from the injection site after each medication event or drug delivery process, and infusion devices or pumping devices with a fluid channel into the body of the patient that remains in use for a prolonged period of time.

Disposable single-use injection devices include auto-injectors and patch injection devices for delivering a fixed or variable dose of drug from a container through a cannula or injection needle. Neither the latter nor the container are intended to be manipulated, let alone replaced, by the user of an auto-injector or patch injection device. Patch injection devices or ready-to-use pre-filled Wearable Bolus Injectors (WBI) or On-Body Delivery Systems (OBDS) are mounted, attached, patched or adhered to the skin of the patient in view of an imminent extended single dose injection taking at least thirty seconds to complete. Patch injection devices were developed for the treatment of diseases like cardiovascular diseases, autoimmune diseases or cancer, and are suitable for the delivery of medicaments which comprise injectable liquids with a high viscosity, and/or which are to be administered in larger single-dose volumes of up to five or even ten milliliters.

Infusion devices include pump devices for continual delivery of a regular or basal amount of medication through a cannula that remains in the injection site for an extended period of time of up to three days. Disposable single-use or semi-disposable infusion devices include patch infusion pumps or wearable basal infusion devices that are mounted, attached, patched or adhered to the skin of the patient in view of an uninterrupted infusion and for delivery of multiple adjustable doses superimposed on a basal rate. The disposable part includes at least a skin-contact area and a cannula that are not intended to be replaced but disposed of after having been in use for a period between thirty minutes and several days. Patch pumps have remote controls and are directly worn on the body, thus eliminating the need for long catheters and direct pump access, and are preliminarily being used for the treatment of diabetes.

EP 3260151 A1 discloses a patch injection device with a bottom housing part, a capacitive sensor, an adhesive patch with a first adhesive layer contacting the bottom housing part and a second adhesive layer to contact the skin of a patient, and a release liner made of coated paper or a thin sheet of plastic that covers the second adhesive layer prior to use of the device. Exemplary sub-assemblies and components of the patch delivery device include a cartridge retainer, a needle insertion mechanism for inserting a cannula into the skin of the patient, a fluid path for connecting the cannula to the cartridge, and a drive means for dispensing the liquid and including a small electric motor and a battery. Patch injection devices are suitable for injectable liquids with a high viscosity of at least 1, preferably at least 15, and most preferably at least 50 cP (0.050 kgm⁻¹s⁻¹).

A widely used type of electric motor is a Permanent Magnet (PM) or Brushless Direct Current (BLDC) motor whereby torque is supplied to the rotating components by imposing an alternating electromagnetic field onto a field generated by a permanent magnet rotor. The absolute angular position of the rotor permanent magnets has to be known for precisely determining the stator current magnitude, measuring the rotor angular position to that end is important. Absolute position sensors such as resolvers and optical encoders are typically used to measure the absolute angular position of the machine rotor. On the other hand, in a sensor-less configuration, no extra rotor position sensor is provided, and the rotation of the rotor is monitored from measurements of a Back Electro-Magnetic Force (Back-EMF) at the terminals of the stator coils.

WO2019/008562 A1 describes a general concept of using back-EMF for controlling the motor of an infusion pump. US 2011/227519 A1 further details such a concept using a "back EMF detection circuit" connected to the poles of the motor coils. In US 2018/069494 A1, the voltage is monitored at the poles of the motor coils and forwarded to a micro-processor for analysis and used to derive information about the movement of the rotor.

US 2008/0197794 A1 describes a general method for sensorless control of a PWM-driven single-phase BLDC motor, where the feedback loop used to control the PWM duty cycle is analysed to extract information about the back EMF force, avoiding an extra circuit for measurement and ensuring full driving power at any time of operation.

Paper DOI: 10.1109/IECON.2005.1569166 "Sensorless control technology for single phase BLDCM based on the winding time-sharing method" from WEIZI WANG ET AL develops a low-cost high-performance sensorless control method for single phase brushless DC motor(BLDCM). It is based on the winding time-sharing principle. During most of time in one control cycle, winding is used as a driving component with load current to drive motor rotating. During the rest of the time, it is used as a sensing component to detect the back EMF.

A preferred single-phase driver circuit connected to terminals of a stator coil of a PM or BLDC motor is implemented with an H-bridge electronic circuit topology. An H-bridge is built with four switches, allowing selectively to apply a positive, or supply, voltage and a negative, or ground, voltage across the motor load. Operating the driver circuit in a high-impedance mode with all four switches being open allows to measure the Back-EMF induced in the stationary coil by the rotating permanent magnets at the coil terminals, and to derive a motor condition or speed therefrom. However, freewheeling diodes generally provided in antiparallel with respective transistor switches of the H-bridge tend to influence, by virtue of their temperature dependent forward blocking voltage, the measurement of the Back-EMF. In addition, at a coil terminal not connected to ground voltage, or connected to ground voltage via a blocking diode, negative voltages down to a blocking voltage of the diode may be observed, which requires a difference amplifier or subtraction stage to establish the difference between the two coil terminal voltages.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

### DESCRIPTION OF THE INVENTION

The invention is defined in the independent claims. Further aspects and preferred embodiments are defined in the dependent claims.

It is an objective of the invention to simplify a sensor-less way of monitoring an electric motor in a drug delivery device, in particular to provide a way of monitoring the electric motor with fewer components. This objective is achieved by a drug delivery device with a motor control unit and by a method of operating a motor control unit of a drug delivery device according to the independent claims. Preferred embodiments are evident from the dependent patent claims.

According to the invention, a drug delivery device for subcutaneous or intra-muscular delivery of a liquid to a patient comprises an electric Brushless Direct Current BLDC motor for driving a piston or stopper in a cartridge to dispense the liquid from the cartridge. The motor is controlled by a motor control unit including a driver circuit with a plurality of switches arranged in H-Bridge topology and alternatingly connecting a first and a second coil terminal of a stator coil of the motor to a supply DC voltage V+ and to ground GND voltage. The motor control unit is adapted to be operated in a motor monitor mode with zero torque applied to a rotor of the motor and allowing a voltage measurement unit to measure a Back Electro-Magnetic Force Back-EMF induced in the stator coil between the first coil terminal and ground GND.

Connecting the second coil terminal to ground GND in the motor monitor mode includes closing or bypassing at least one switch of the driver circuit. The Back-EMF as the voltage difference between the two coil terminals being available at the first coil terminal obviates the need for a difference circuit or amplifier to subtract a first from a second terminal voltage, and thus results in a simplified control circuitry with less components. Any diode connected in antiparallel to the at least one switch is likewise bypassed, which eliminates a possibly temperature-dependent contribution to the Back-EMF on the part of the diode. The Back-EMF measured in this manner may be exploited in view of monitoring a speed, condition or status of the electric motor in a sensor-less way excluding the presence of any extra or dedicated rotor position sensor.

In a preferred variant of the invention, the driver circuit is adapted to be controlled, in the monitor mode, to close the at least one switch of the H-Bridge in order to connect the second coil terminal to ground GND, and to open the remaining switches of the driver circuit. Such a modified high-impedance mode is specifically suitable for operating a driver circuit with switch components for which such individual controllability is not limited by operational safety constraints.

In an advantageous embodiment of the invention, the driver circuit is operated, in the monitor mode, in a high-impedance mode with all four switches being open, and the second coil terminal is connected to ground GND via a ground switch of the motor control unit. The ground switch is external to the driver circuit, either embedded in a driver control circuit or provided as a separate switch element of the motor control unit. The ground switch is specifically suitable in connection with an integrated driver circuit with limited individual controllability of the transistor switches.

In a further preferred embodiment, the drug delivery device is a patch injection device or a wearable bolus injector for delivering a single dose or bolus volume from a reservoir through a single use cannula when/upon adhering to the skin of a patient. Patch injection devices dispense a single dose in a single piston stroke taking at least thirty seconds and preferably between five and thirty minutes. As the piston or stopper is in any case driven to the very end of the reservoir, precise knowledge of the stopper position may not be required, and a motor monitoring activity may be limited to confirming that the rotor is rotating at all. Preferably, in the case of a patch injection device, the rotor speed of the electric motor does not exceed 50 Hz, and measurement of the Back-EMF occurs four times or less per cycle and per motor phase. The modest number of individual successive Back-EMF measurements obtained this way is sufficient to establish a basic monitoring of the motor operation without requiring undue processing or computational power.

Alternatively, the drug delivery device may be a disposable patch infusion pump device, or a reusable infusion pump device with flexible catheter tubing. However, for such basal-type pump devices, the presence of a separate motor sensor may be unavoidable in order to guarantee with highest certainty that motor activity and incremental piston movements confirm to the expected.

The invention is also directed to a motor control unit for an electric motor with a stator coil, the motor control unit including a driver circuit with a plurality of switches alternatingly connecting a first and a second coil terminal of the stator coil of the electric motor to supply voltage (V+) and to ground (GND), wherein the motor control unit is adapted to be operated in a monitor mode for measurement of a Back Electro-Magnetic Force (Back-EMF) of the stator coil between the first coil terminal and ground (GND).

### BRIEF DESCRIPTION OF THE DRAWINGS

The subject matter of the invention will be explained in more detail in the following text with reference to preferred exemplary embodiments which are illustrated in the attached drawings, in which:
Fig.1 shows a lateral view and a top view of a patch delivery device;
Fig.2 depicts an exploded view of a drive sub-assembly of the patch delivery device;
Fig.3 depicts a single-phase driver circuit of a motor controller of a BLDC motor;
Fig.4 depicts an equivalent configuration of a prior-art Back-EMF measurement;
Fig.5 depicts an equivalent configuration of a Back-EMF measurement according to an embodiment of the invention,
Fig.6 depicts a motor control unit with driver circuit and a driver control circuit, and
Fig.7 depicts a motor control unit with driver circuit and an alternative driver control circuit.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

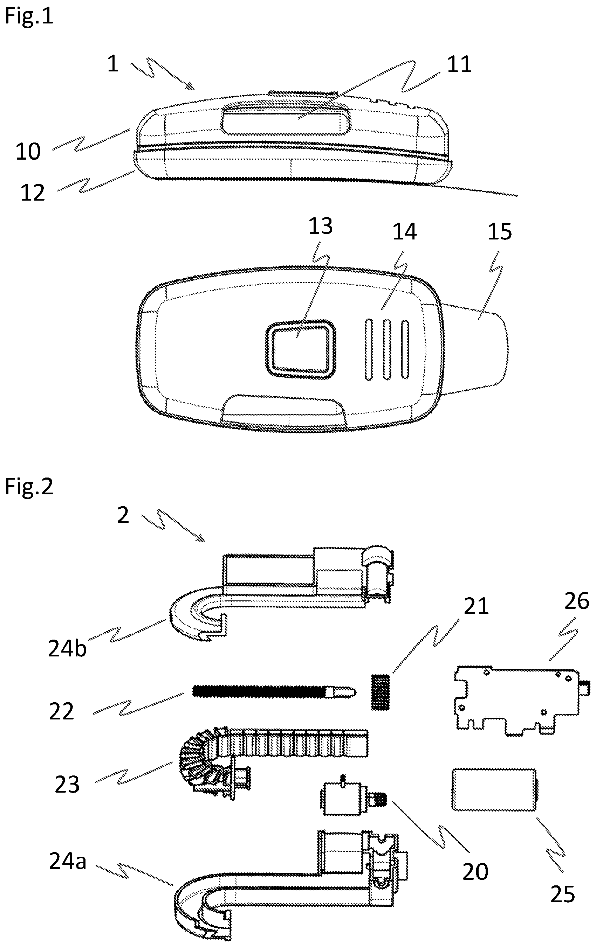

Fig.1 includes a lateral view (top half) and a top view (bottom half) of a patch delivery device comprising a housing 1 with a cover housing part 10 including a window 11 for viewing a cartridge, a bottom housing part or base plate 12, a release button 13 for activating the device and/or starting the injection, and an optical indicator 14 such as an LED. A patient facing side or skin contact area of the bottom housing part is to be adhered or attached to the skin of a patient by means of an adhesive patch, of which a release liner lid 15 laterally extends beyond the skin contact area. Exemplary sub-assemblies and components of the patch delivery device include a cartridge retainer for holding a cartridge or reservoir with a liquid medicament to be dispensed by a moving piston, stopper, or bung; a needle insertion mechanism for inserting a cannula or a hollow needle into the skin of the patient; a fluid path for connecting the cannula to the cartridge; and a drive means for driving the needle insertion mechanism. These sub-assemblies are amply described for instance in EP 3260151 A1.

Fig.2 depicts components of a piston drive sub-assembly 2 for dispensing the liquid medicament from the cartridge, including an electric motor 20 driving a gear wheel 21 axially and rotationally fixed to a threaded rod 22. The threaded rod 22 has an external threading for engaging an internal threading at the proximal end of a non-rotating piston rod 23 such that a rotating movement of the former is transferred to a linear movement of the latter. A drive carrier 24a and cover 24b hold and guide in a U-turn the flexibly connected segments of the piston rod 23. A distal end of the piston rod 23 urges the piston or stopper in the cartridge towards the cannula to cause medication to be dispensed from the cartridge. A power source 25 and a motor control unit 26 for operating the electric motor 20 are likewise part of the subassembly, wherein the power source may be a battery with a DC supply voltage V+ of 3.3 Volts, and wherein the control unit may include circuitry and components arranged on a Printed Circuit Board (PCB).

The needle insertion mechanism for insertion of an injection needle or cannula into the skin of the patient may additionally be equipped with a needle retraction feature, to retract the injection needle or cannula into the device before the device is being removed from the injection site. A needle insertion mechanism may either insert first a needle together with a soft cannula surrounding the needle, wherein the needle is retracted prior to delivery of the medication through the soft cannula, or a hollow injection needle or rigid cannula made e.g. of steel is inserted in the tissue and also used for subsequent delivery. The insertion mechanism for inserting the delivery element may include an electric motor and a gear mechanism, which motor may or may not be identical with the piston driving motor 22.

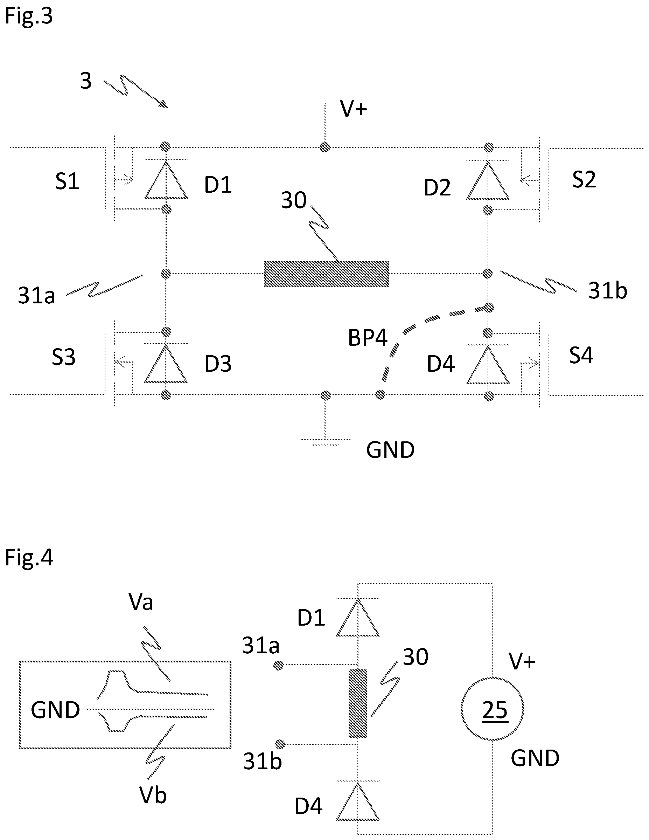

Fig.3 depicts a single-phase driver circuit 3 for a Brush-Less Direct Current (BLDC) motor coil 30 according to a first embodiment. Coil terminals 31a, b of the motor coil 30 are connected at the center of an H-bridge circuit that includes solid-state, or semiconductor, switches S1-S4 and integrated power diodes D1-D4. The switches S1-S4 include p- or n-channel Field Effect Transistors controlled by control signals applied to the transistor gates and prepared by a driver control circuit. Each of the diodes D1-D4 is provided in antiparallel with a respective switch S1-S4. The H-bridge is also connected to supply voltage V+ and to ground GND voltage. Current is passed through the motor coil in a desired direction by controlling the on/off status of the switches S1-S4.

Operating the driver circuit 3 in a high-impedance, zero-torque mode with all four switches S1-S4 being open for short periods of time allows to measure the Back Electro-Magnetic Force (Back-EMF) induced in the stator coil 30 by the rotating permanent magnets. To that purpose a voltage measurement unit of the motor control unit 26 may be connected to the coil terminals 31a, 31b, or the terminal voltages at the two coil terminals 31a, 31b may be individually determined against ground and a value of the Back-EMF derived therefrom. However, the diodes D3, D4 connected in antiparallel with a respective transistor switch S3, S4 connected to ground tend to delay and bias, by virtue of their temperature dependence and limited forward blocking voltage, the establishment of the high-impedance state.

Fig.4 depicts schematically the equivalent configuration in this case, with an evolution of the terminal voltages Va and Vb in a motor monitor mode being shown in the insert. The initial plateau and decay are due to transient currents and diode effects. Subsequently, the terminal voltages reach a steady state with the alternating terminal voltages being almost constant as the interval depicted is less than one percent of the rotor period. It is apparent that in the steady state a negative voltage Vb is registered at terminal 31b, which necessitates the use of a difference circuit to establish the Back EMF between the two coil terminals.

According to the invention, the second coil terminal 31b is connected to ground voltage GND in view of and during Back-EMF measurement step. This is represented by the curved broken line BP4 bypassing switch S4 and diode D4 in Fig.3. Alternatively or alternatingly, the Back-EMF may be measured while connecting terminal 31a to ground. To that end, in an embodiment of the invention, switch S4 is closed in the monitor mode.

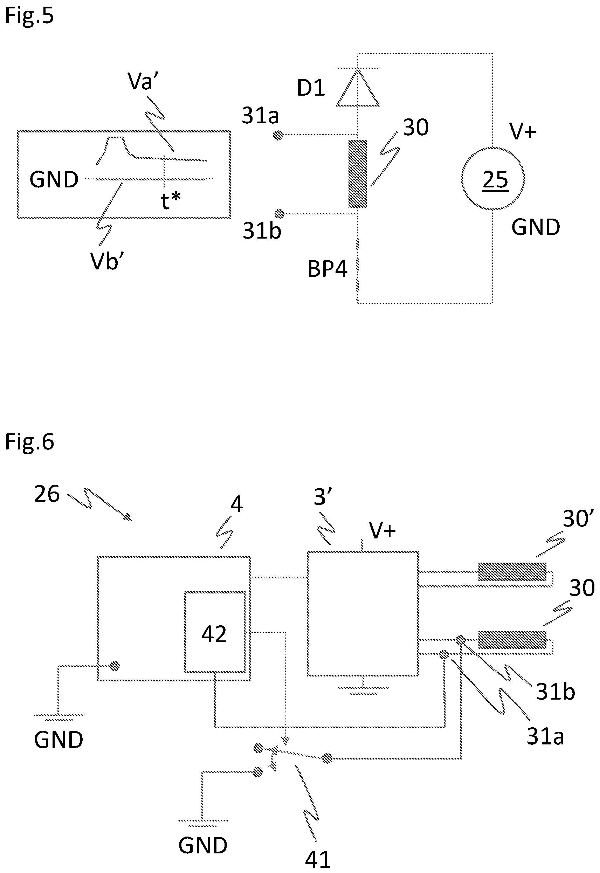

Fig.5 depicts schematically the equivalent configuration in the case of the invention, with an evolution of the terminal voltages Va' and Vb' during motor monitor mode being shown in the insert. The initial plateau and decay of terminal voltage Va' at terminal 31a are due to transient currents and diode effects, while terminal voltage Vb' at terminal 31b is locked to ground potential GND. In the steady state the terminal voltage Va' alone is representative of, or even identical to, the Back-EMF between the two coil terminals. The alternating terminal voltage is almost constant, as the interval depicted in the insert is again far less than one percent of the rotor period. Accordingly, the actual measurement of the terminal voltage value may take place at any instant t* after the transient effects have faded. A non-zero value of the Back-EMF may already be indicative of a rotating motor and/or the absence of rotor blockage or the like. Preferably, the measurement instant t* may be chosen at the end of a fixed time interval after entering the motor monitor mode. This allows for a reasonable comparison of successive measurements of the Back-EMF, from which in turn a coarse information about an evolution of a motor operation may be derived.

The monitor mode in which the Back-EMF is measured is based on a conventional high-impedance mode with all four bridge transistor switches being open. Accordingly, no energy from the power source is used to build up a torque on the rotor while the control unit is in the monitor mode. Ground voltage (GND) designates the voltage level of the motor control unit that is common to the battery and to the voltage measurement unit, and does not necessarily imply a connection to Earth.

By way of example, the measurement instant t* may be chosen between 0.1 and 1 ms, and preferably about 0.2 ms, after establishment of the high-impedance mode, with the preferred value being equal to one percent of the shortest rotor period in patch injection devices. The measurement of the Back-EMF is preferably initiated at a constant step of the driving cycle, such as at zero crossings of a sinusoidal applied or driving voltage, and therefore occurs at the rotor frequency or multiples thereof.

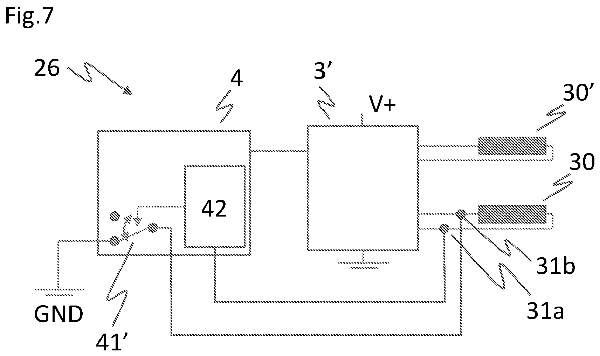

Fig.6 and Fig.7 depict an exemplary motor control unit 26 for a two-phase electric motor with two stator coils 30, 30', including a motor driver circuit 3 and a driver control circuit 4. The driver control circuit 4 is a microcontroller including logic for preparing control or gate signals on behalf of the driver circuit 3. The latter supplies an output current to stator coil terminals 31a, 31b of the first stator coil 30 as well as to the second stator coil 30', and is either realized with discrete electronic components or an integrated circuit including the transistor switches. State of the art integrated driver circuits suitable for driving BLDC motors however may embed safety logic that in particular prevents the transistors from being closed or opened in certain circumstances. Accordingly, control signals from the driver control circuit 4 aiming at establishing a modified high-impedance state as described above may be ignored, and the diode bypass state according to the invention may have to be established outside the driver circuit 3.

To that end, a second terminal 31b of the stator coil 30 is connected, via circuitry or conductor paths entirely outside the driver circuit 3, to a ground switch 41, 41' controllable to connect the circuitry and hence the stator terminal to ground potential GND. As depicted in Fig.6, the ground switch 41 may be a dedicated switch of the motor control unit 26, and provided on a same PCB supporting the driver circuit and the driver control circuit. Alternatively, as depicted in Fig.7, a switch 41' of the microprocessor integrating the driver control circuit 4 may be used to this purpose. The driver control circuit 4 of Fig.6 and Fig.7 further includes a voltage measurement unit 42 with an analog-to-digital converter, or a sample and hold circuit, that is connected to a first terminal 31a and configured to measure and record a terminal voltage Va of the stator coil 30 with respect to ground GND. The voltage measurement unit 42 may be configured to control the ground switch 41, 41'.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: Device housing
- 10: Cover housing part
- 11: Window
- 12: Bottom housing part
- 13: Release button
- 14: Optical indicator
- 15: Release liner lid
- 2: Piston drive sub-assembly
- 20: Electromotor
- 21: Gear wheel
- 22: Threaded rod
- 23: Piston rod
- 24a, b: Drive carrier, cover
- 25: Battery
- 26: Motor control unit
- 3: Driver circuit
- 30: Stator coil
- 31a, b: Coil terminals
- 4: Driver control circuit
- 41, 41': Ground switch
- 42: Voltage measurement unit

## Claims

1. A drug delivery device with
an electric motor (20) for driving a piston (23) in a cartridge to dispense a liquid from the cartridge,
a motor control unit (26) including
a driver circuit (3) with a H-bridge arrangement of switches (S1 to S4) alternatingly connecting a first and a second coil terminal (31a, 31b) of a stator coil (30) of the motor to supply voltage (V+) and to ground (GND),
and a voltage measurement unit (42) configured to measure a Back Electro-Magnetic Force (Back-EMF) of the stator coil (30) between the first coil terminal (31a) and ground (GND),
whereby the motor control unit (26) is adapted to
drive the motor while periodically switching to a monitor mode with zero torque applied to a rotor of the motor,
and to measure the Back-EMF in said monitor mode,
***characterised* in that** the driver circuit (3) is adapted to be controlled, in the monitor mode, to close one switch (S4) in order to connect the second coil terminal (31b) to ground while opening the remaining three switches.

2. The drug delivery device of claim 1, **characterized in that** the motor control unit includes a ground switch (41, 41'), and **in that** in the monitor mode, the driver circuit (3') is operated in a high-impedance mode with all four switches (S1 - S4) being open, and the second coil terminal (31b) is connected to ground GND via the ground switch.

3. The drug delivery device of one of the preceding claims, **characterized in that** the device is a patch injection device or a wearable bolus injector with a bottom housing part (12) to be adhered to the skin of a patient.

4. The drug delivery device of claim 3, **characterized in that** the rotor speed of the electric motor does not exceed 50 Hz, preferably 25 Hz, and that measurement of the Back-EMF occurs four times or less, in particular twice, per cycle and per motor phase.

5. The drug delivery device of one of claims 1 or 2, **characterized in that** the device is a disposable patch infusion pump device or a reusable infusion pump device with catheter tubing.

6. A method of measuring a Back Electro-Magnetic Force (Back-EMF) by operating a motor control unit (26) of a drug delivery device with an electric motor (20) for driving a piston (23) in a cartridge of the drug delivery device to dispense a liquid from the cartridge, the motor control unit (26) including
a driver circuit (3) with a H-bridge arrangement of switches (S1 to S4) alternatingly connecting a first and a second coil terminal (31a, 31b) of a stator coil (30) of the motor to supply voltage (V+) and to ground (GND),
and a voltage measurement unit (42) configured to measure a Back Electro-Magnetic Force (Back-EMF) of the stator coil (30) between the first coil terminal (31a) and ground (GND),
the method comprising
- driving the motor while periodically switching to a monitor mode with zero torque applied to a rotor of the motor
- connecting a voltage measurement unit (42) between the first coil terminal (31a) and ground (GND),
- connecting the second coil terminal (31b) to ground (GND) by closing one switch (S4) while opening the remaining three switches (S1 to S3) of the driver circuit (3) and
- measuring a Back Electro-Magnetic Force (Back-EMF) of the stator coil (30) by the voltage measurement unit (42).

7. The method of claim 6, comprising
- connecting the second coil terminal (31b) to ground (GND) via a ground switch (41, 41') of the motor control unit (26), and
- operating the driver circuit (3') in a high-impedance mode with all four switches (S1 - S4) being open.

## Patentansprüche

1. Arzneimittelabgabevorrichtung mit
einem elektrischen Motor (20) zum Antreiben eines Kolbens (23) in einer Kartusche, um eine Flüssigkeit aus der Kartusche zu dispensieren,
einer Motorsteuerungseinheit (26) einschließlich
einer Treiberschaltung (3) mit einer H-Brückenanordnung von Schaltern (S1 bis S4), die einen ersten und einen zweiten Spulenanschluss (31a, 31b) einer Statorspule (30) des Motors mit einer Versorgungsspannung (V+) und einer Masse (GND) abwechselnd verbindet,
und einer Spannungsmesseinheit (42), die konfiguriert ist, um eine gegenelektromotorische Kraft (Gegen-EMF) der Statorspule (30) zwischen dem ersten Spulenanschluss (31a) und der Masse (GND) zu messen,
wobei die Motorsteuerungseinheit (26) angepasst ist zum
Antreiben des Motors unter periodischem Umschalten in einen Überwachungsmodus, in dem auf einen Rotor des Motors ein Drehmoment von null ausgeübt wird,
und Messen der Gegen-EMK in dem Überwachungsmodus,
***dadurch gekennzeichnet, dass*** die Treiberschaltung (3) angepasst ist, um in dem Überwachungsmodus gesteuert zu werden, um einen Schalter (S4) zu schließen, um den zweiten Spulenanschluss (31b) unter Öffnen der übrigen drei Schalter mit der Masse zu verbinden.

2. Medikamentenabgabevorrichtung nach Anspruch 1,
**dadurch gekennzeichnet, dass** die Motorsteuerungseinheit einen Masseschalter (41, 41') einschließt, und dadurch, dass in dem Überwachungsmodus die Treiberschaltung (3') in einem hochohmigen Modus betrieben wird, in dem alle vier Schalter (S1 - S4) offen sind und der zweite Spulenanschluss (31b) über den Masseschalter mit der Masse GND verbunden ist.

3. Arzneimittelabgabevorrichtung nach einem der vorstehenden Ansprüche, **dadurch gekennzeichnet, dass** die Vorrichtung eine Patch-Injektionsvorrichtung oder ein tragbarer Bolusinjektor mit einem unteren Gehäuseteil (12) ist, das auf die Haut eines Patienten zu kleben ist.

4. Medikamentenabgabevorrichtung nach Anspruch 3,
**dadurch gekennzeichnet, dass** die Drehzahl des Rotors des elektrischen Motors 50 Hz, vorzugsweise 25 Hz, nicht überschreitet, und dass die Messung der Gegen-EMK viermal oder weniger, insbesondere zweimal, pro Zyklus und pro Motorphase erfolgt.

5. Medikamentenabgabevorrichtung nach einem der Ansprüche 1 oder 2, **dadurch gekennzeichnet, dass** die Vorrichtung eine wegwerfbare Patch-Infusionspumpenvorrichtung oder eine wiederverwendbare Infusionspumpenvorrichtung mit Katheterschlauch ist.

6. Verfahren zum Messen einer gegenelektromotorischen Kraft (Gegen-EMF) durch Betreiben einer Motorsteuerungseinheit (26) einer Medikamentenabgabevorrichtung mit einem elektrischen Motor (20) zum Antreiben eines Kolbens (23) in einer Kartusche der Medikamentenabgabevorrichtung, um eine Flüssigkeit aus der Kartusche zu dispensieren, wobei die Motorsteuerungseinheit (26) einschließt
eine Treiberschaltung (3) mit einer H-Brückenanordnung von Schaltern (S1 bis S4), die einen ersten und einen zweiten Spulenanschluss (31a, 31b) einer Statorspule (30) des Motors mit der Versorgungsspannung (V+) und mit der Masse (GND) abwechselnd verbindet,
und eine Spannungsmesseinheit (42), die konfiguriert ist, um eine gegenelektromotorische Kraft (Gegen-EMF) der Statorspule (30) zwischen dem ersten Spulenanschluss (31a) und der Masse (GND) zu messen,
das Verfahren umfassend
- Antreiben des Motors unter periodischem Umschalten in einen Überwachungsmodus, in dem ein Drehmoment von null auf einen Rotor des Motors ausgeübt wird
- Verbinden einer Spannungsmesseinheit (42) zwischen dem ersten Spulenanschluss (31a) und der Masse (GND),
- Verbinden des zweiten Spulenanschlusses (31b) mit der Masse (GND) durch Schließen eines Schalters (S4) unter Öffnen der übrigen drei Schalter (S1 bis S3) der Treiberschaltung (3) und
- Messen einer gegenelektromotorischen Kraft (Gegen-EMK) der Statorspule (30) durch die Spannungsmesseinheit (42).

7. Verfahren nach Anspruch 6, umfassend
- Verbinden des zweiten Spulenanschlusses (31b) mit der Masse (GND) über einen Masseschalter (41, 41') der Motorsteuerungseinheit (26) und
- Betreiben der Treiberschaltung (3') in einem hochohmigen Modus, in dem alle vier Schalter (S1 - S4) offen sind.

## Revendications

1. Dispositif d'administration de substance médicamenteuse avec
un moteur électrique (20) destiné à entraîner un piston (23) dans une cartouche pour distribuer un liquide à partir de la cartouche,
une unité de commande de moteur (26) comportant
un circuit d'attaque (3) avec un agencement en pont en H de commutateurs (S1 à S4) connectant alternativement une première et une seconde borne de bobine (31a, 31b) d'une bobine de stator (30) du moteur à une tension d'alimentation (V+) et à la terre (GND),
et une unité de mesure de tension (42) configurée pour mesurer une contre force électromagnétique (contre-EMF) de la bobine de stator (30) entre la première borne de bobine (31a) et la terre (GND),
moyennant quoi l'unité de commande de moteur (26) est adaptée pour
entraîner le moteur tout en commutant périodiquement vers un mode de surveillance avec un couple nul appliqué à un rotor du moteur,
et pour mesurer la contre-EMF dans ledit mode de surveillance,
***caractérisé* en ce que** le circuit d'attaque (3) est adapté pour être commandé, dans le mode surveillance, pour fermer un commutateur (S4) afin de connecter la seconde borne de bobine (31b) à la terre tout en ouvrant les trois commutateurs restants.

2. Dispositif d'administration de substance médicamenteuse selon la revendication 1, **caractérisé en ce que** l'unité de commande de moteur comporte un commutateur de terre (41, 41'), et **en ce que**, dans le mode surveillance, le circuit d'attaque (3') est amené à fonctionner dans un mode à haute impédance avec les quatre commutateurs (S1 à S4) ouverts, et la seconde borne de bobine (31b) est connectée à la terre GND par l'intermédiaire du commutateur de terre.

3. Dispositif d'administration de substance médicamenteuse selon l'une des revendications précédentes, **caractérisé en ce que** le dispositif est un dispositif d'injection sur timbre ou un injecteur en bolus à porter sur soi avec une partie de boîtier inférieur (12) destinée à être fixée par adhérence à la peau d'un patient.

4. Dispositif d'administration de substance médicamenteuse selon la revendication 3, **caractérisé ence que** la vitesse de rotor du moteur électrique ne dépasse pas 50 Hz, de préférence 25 Hz, et qu'une mesure de la contre-EMF a lieu quatre fois ou moins, en particulier deux fois, par cycle et par phase de moteur.

5. Dispositif d'administration de substance médicamenteuse selon l'une des revendications 1 ou 2, **caractérisé en ce que** le dispositif est un dispositif de pompe à perfusion sur timbre jetable ou un dispositif de pompe à perfusion réutilisable avec une tubulure de cathéter.

6. Procédé de mesure d'une contre force électromagnétique (contre-EMF) en faisant fonctionner une unité de commande de moteur (26) d'un dispositif d'administration de substance médicamenteuse avec un moteur électrique (20) pour entraîner un piston (23) dans une cartouche du dispositif d'administration de substance médicamenteuse pour distribuer un liquide à partir de la cartouche, l'unité de commande de moteur (26) comportant
un circuit d'attaque (3) avec un agencement en pont en H de commutateurs (S1 à S4) connectant alternativement une première et une seconde borne de bobine (31a, 31b) d'une bobine de stator (30) du moteur à une tension d'alimentation (V+) et à la terre (GND),
et une unité de mesure de tension (42) configurée pour mesurer une contre force électromagnétique (contre-EMF) de la bobine de stator (30) entre la première borne de bobine (31a) et la terre (GND),
le procédé comprenant
- l'entraînement du moteur tout en commutant périodiquement vers un mode de surveillance avec un couple nul appliqué à un rotor du moteur
- la connexion d'une unité de mesure de tension (42) entre la première borne de bobine (31a) et la terre (GND),
- la connexion de la seconde borne de bobine (31b) à la terre (GND) en fermant un commutateur (S4) tout en ouvrant les trois commutateurs restants (S1 à S3) du circuit d'attaque (3) et
- la mesure d'une contre force électromagnétique (contre-EMF) de la bobine de stator (30) par l'unité de mesure de tension (42).

7. Procédé selon la revendication 6, comprenant
- la connexion de la seconde borne de bobine (31b) à la terre (GND) par l'intermédiaire d'un commutateur de terre (41, 41') de l'unité de commande de moteur (26), et
- le fonctionnement du circuit d'attaque (3') dans un mode à haute impédance avec les quatre commutateurs (S1 à S4) ouverts.
